(19) Europäisches Patentamt
European Patent Office
Office européen des brevets

(11) Veröffentlichungsnummer: **0 462 452 A1**

# (12) EUROPÄISCHE PATENTANMELDUNG

(21) Anmeldenummer: **91109245.0**

(51) Int. Cl.5: **C07D 239/28**, C07D 239/30

(22) Anmeldetag: **06.06.91**

(30) Priorität: **19.06.90 DE 4019420**

(43) Veröffentlichungstag der Anmeldung:
**27.12.91 Patentblatt 91/52**

(84) Benannte Vertragsstaaten:
**CH DE FR GB LI**

(71) Anmelder: **BAYER AG**

**W-5090 Leverkusen 1 Bayerwerk(DE)**

(72) Erfinder: **Beck, Gunther, Dr.**
**Am Mittelberg 19**
**W-5090 Leverkusen(DE)**
Erfinder: **Kysela, Ernst, Dr.**
**Virchowstrasse 14**
**W-5060 Bergisch Gladbach(DE)**

## (54) Polycyanopyrimidine und Verfahren zu ihrer Herstellung.

(57) Die Erfindung betrifft neue Polycyanopyrimidine der Formel

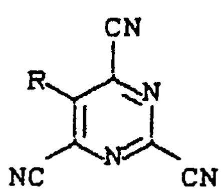

(I) ,

in der
R für Chlor oder Cyano steht,
und ein Verfahren zu ihrer Herstellung.

EP 0 462 452 A1

Die Erfindung betrifft neue Polycyanopyrimidine und ein Verfahren zu ihrer Herstellung.

Es wurden neue Polycyanopyrimidine der Formel

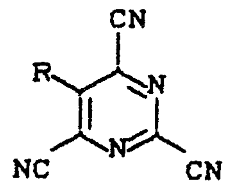

(I)

gefunden, in der

R für Chlor oder Cyano steht.

Die Erfindung betrifft ferner ein Verfahren zur Herstellung der Polycyanopyrimidine der Formel (I); das Verfahren ist dadurch gekennzeichnet, daß man Polyfluoropyrimidine der Formel

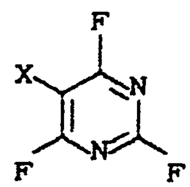

(II),

in der

X für Chlor oder Fluor steht,

mit Alkalicyaniden in organischen Verdünnungsmitteln umsetzt.

Als Alkalicyanide werden vorzugsweise die wohlfeilen Cyanide des Natriums oder Kaliums verwendet.

Die Umsetzung mit den Alkalicyaniden wird in organischen Verdünnungsmitteln vorgenommen. Als organische Verdünnungsmittel werden übliche aprotische polare bzw. dipolare Lösungsmittel eingesetzt. Beispiele für solche Lösungsmittel sind:

Aliphatische Nitrile wie Acetonitril, Propionitril, 3-Methoxy-propionitril; aliphatische und cyclische Ether wie Ethylenglykoldimethylether, Diethylenglykoldimethylether (Diglyme), Tetrahydrofuran; N,N-Dialkylamide niederer aliphatischer Carbonsäuren, wie N,N-Dimethylformamid, N,N-Dimethylacetamid, N-Methylpyrrolidon; aliphatische Sulfoxide wie Dimethylsulfoxid; aliphatische Sulfone wie Dimethylsulfon und Tetramethylensulfon; ferner Tetramethylharnstoff, N,N-Dimethylmethyl-1,3-imidazolidin-2-on und Hexamethylphosphorsäuretrisamid. Besonders bevorzugt sind Acetonitril und N,N-Dimethylformamid.

Die Umsetzung der Polyfluoropyrimidine der Formel (22) mit den Alkalicyaniden kann bei Temperaturen von -70°C bis +50°C, vorzugsweise von -50°C bis +30°C, besonders bevorzugt von -40°C bis +10°C ausgeführt werden.

Die Reaktion wird im allgemeinen bei Normaldruck vorgenommen.

Die erfindungsgemäße Umsetzung der Verbindungen der Formel (II) mit den Alkalicyaniden läßt sich durch folgende Reaktionsgleichungen beschreiben:

a) bei der Umsetzung zu 5-Chloro-2,4,6-tricyanopyrimidin:

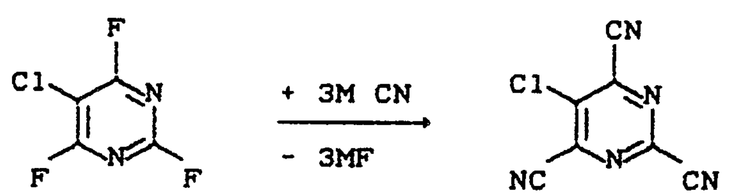


(M = Alkalimetall)

b) bei der Umsetzung zu Tetracyanopyrimidin:

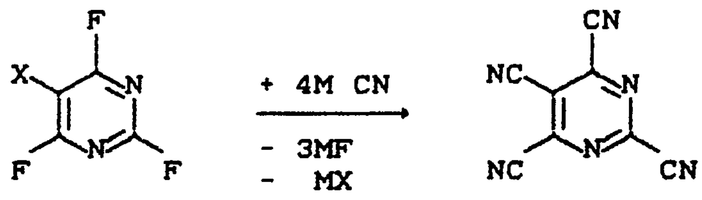


(X = Cl oder F)

Wie aus den vorstehenden Reaktionsgleichungen (a) und (b) hervorgeht, sind zur Herstellung der erfindungsgemäßen Polycyanopyrimidine der Formel (I) 3 oder 4 Mol Alkalicyanid je Mol Ausgangsverbindung (II) erforderlich.

Zur Herstellung von 5-Chloro-2,4,6-tricyano-pyrimidin mit möglichst geringen Gehalten an Tetracyanopyrimidin dürfen höchstens 3 Mol Alkalicyanid je Mol 5-Chloro-2,4,6-trifluoro-pyrimidin verwendet werden. Bei Verwendung des besonders bevorzugten Lösungs- bzw. Verdünnungsmittels Dimethylformamid ist die Umsetzung im Temperaturbereich von -40°C bis -20°C besonders bevorzugt.

Es wurde gefunden, daS es vorteilhaft sein kann, mit einem Unterschuß an Alkalicyaniden zu arbeiten. Die erfindungsgemäße Umsetzung der Polyfluoropyrimidine der Formel (II) mit den Alkalicyaniden verläuft zwar, wie man bei der gaschromatografischen Verfolgung der Umsetzung feststellen kann, über die Zwischenstufen isomerer Monocyano-trihalogen-pyrimidine, z.B. bei Verwendung des 5-Chloro-2,4,6-trifluoropyrimidins als Verbindung (II) über 5-Chloro-4-cyano-2,6-difluoro-pyrimidin und 5-Chloro-2-cyano-4,6-difluoro-pyrimidin, und Dicyano-dihalogen-pyrimidine, z.B. bei Verwendung des 5-Chloro-2,4,6-trifluorpyrimidins als Polyfluoropyrimidin der Formel (II) 5-Chloro-2,4-dicyano-6-fluoro-pyrimidin und z.B. bei Verwendung von Tetrafluoropyrimidin als Polyfluoropyrimidin der Formel (II) 2,4-Dicyano-5,6-difluoro-pyrimidin. Die beiden letztgenannten Dicyanodihalogen-pyrimidine sind sogar durch fraktionierte Sublimation isolierbar. Trotzdem entstehen auch bei Verwendung eines Cyanid-Unterschusses im wesentlichen nur die erfindungsgemäßen Polycyanpyrimidine der Formel (I), und es bleibt eine dem Cyanid-Unterschuß entsprechende Menge an unumgesetzten Ausgangspyrimidin der Formel (II) zurück.

Zur Herstellung von Tetracyanopyrimidin mit möglichst geringem Gehalt an 5-Chloro-2,4,6-tricyanopyrimidin aus dem bevorzugt als Ausgangsprodukt verwendeten 5-Chloro-2,4,6-trifluoro-pyrimidin (II, X = Cl), werden vorzugsweise 4 Mol Alkalicyanid je Mol 5-Chloro-2,4,6-trifluoro-pyrimidin eingesetzt; außerdem hat sich bei Verwendung des besonders bevorzugten Verdünnungsmittels Dimethylformamid ein Temperaturbereich von 0°C bis 20°C besonders bewährt. Arbeitet man dagegen z.B. bei Temperaturen zwischen -30°C und 0°C, erhält man bei Reaktionszeiten zwischen etwa 1 bis 10 Stunden Gemische aus 5-Chloro-2,4,6-tricyano-pyrimidin und Tetracyanopyrimidin, die jedoch nach in der organischen Chemie üblichen Methoden, z.B. durch Chromatographie, durch Umkristallisation oder durch fraktionierte Sublimation in ihre Einzelbestandteile aufgetrennt werden können.

Zur Herstellung von Tetracyanopyrimidin aus dem schwieriger zugänglichen und deshalb nicht bevorzugten Ausgangsprodukt Tetrafluoropyrimidin (II, X = F) arbeitet man, insbesondere auch im Hinblick auf dessen besonders hohe Reaktivität, besonders bevorzugt im unteren Teil des angegebenen Temperaturbereichs. Die Verwendung eines Unterschusses (z.B. nur 50 % der stöchiometrisch erforderlichen Menge) an Alkalicyanid ist in diesem Falle problemlos, da gegebenenfalls mit entstandenes 2,4-Dicyano-5,6-difluoropyrimidin infolge seiner Leichtflüchtigkeit z.B. durch fraktionierte Sublimation leicht abgetrennt werden kann.

Die bei Anwendung eines Cyanidunterschusses und/oder vorzeitig abgebrochener Reaktion in dem Reaktionsgemisch unumgesetzt vorliegenden Ausgangsprodukte der Formel (II) sind wegen ihrer verglichen mit den erfindungsgemäßen Polycyanopyrimidinen der Formel (I) erheblich leichteren Flüchtigkeit leicht abtrennbar und können im nächsten Ansatz wiederverwendet werden.

Es wurde gefunden, daß man die erfindungsgemäßen Polycyanopyrimidine der Formel (I) nur dann in mittleren bis guten Ausbeuten erhält, wenn man die Polyfluoropyrimidine der Formel (II) mit Alkalicyaniden unter milden Reaktionsbedingungen umsetzt und für die Umsetzung Alkalicyanide höchstens in einem geringeren Überschuß über die stöchiometrisch erforderliche Menge anwendet. Durch die Parameter "milde Reaktionsbedingungen " und "höchstens geringer Überschuß an Alkalicyaniden" wird erreicht, daß die Bildung unerwünschter Folgeprodukte, die aufgrund der hohen Reaktionsfähigkeit der Polycyanopyrimidine der Formel (I), insbesondere des Tetracyanopyrimidins, im Vordergrund steht, so weit unterbunden wird, daß die gewünschten Polycyanopyrimidine der Formel (I) noch im mittleren bis guten Ausbeuten erhalten werden.

Die Erfindung betrifft daher im besonderen ein Verfahren zur Herstellung von Polycyanopyrimidinen der Formel (I), das dadurch gekennzeichnet ist, daß man Polyfluoropyrimidine der Formel (II) in organischen Verdünnungsmitteln unter milden Reaktionsbedingungen mit höchstens einem Überschuß von 20 %, vorzugsweise höchstens einem Überschuß von 10 % über die stöchiometrisch erforderliche Menge an Alkalicyaniden umsetzt.

Die Ausgangsprodukte der Formel (II), 5-Chloro-2,4,6-trifluoro-pyrimidin und Tetrafluoropyrimidine, sind wohlbekannte Verbindungen, die z.T. in industriellem Maßstab produziert werden.

Die erfindungsgemäße Umsetzung der Alkalicyanide mit den Verbindungen der Formel (II) wird vorzugsweise so ausgeführt, daß man die Lösung des Polyfluoropyrimidins der Formel (II) im betreffenden wasserfreien Lösungsmittel unter Feuchtigkeitsauschluß und unter Rühren unter milden Bedingungen, d.h. bei Temperaturen von -50°C bis -40°C mit der stöchiometrisch erforderlichen Menge Alkalicyanid (oder einem Unterschuß) versetzt und bei dem gewählten Endtemperaturbereich von -30°C bis +30°C z.B. 1- 10 Stunden kräftig weiterrührt. Nach beendeter (oder vorzeitig abgebrochener) Umsetzung wird die Reaktionsmischung im allgemeinen in überschüssiges (z.B. das zwei- bis 10-fache Volumen) Eiswasser gegeben, das mindestens so viel Mineralsäure, z.B. Salzsäure, enthält, daß nach Einrühren der Reaktionslösung der pH-Wert <7 ist. Anschließend werden die erfindungsgemäßen Polycyanopyrimidine der Formel (I) durch Filtration isoliert und gegebenenfalls nachgereinigt.

Polycyanobenzole und Polycyano(poly-)aza-benzole sind wichtige Ausgangsmaterialien für die Herstellung von Charge-Transfer-Komplexen; diese CT-Komplexe zeichnen sich z.T. durch eine sehr gute elektrische Leitfähigkeit aus (s. z.B. Bull. Acad. Polon. Sci. 23, 563 (1975); Dissertation von Peter Neumann, Universität Freiburg i. Breisgau, BRD, 1968; Synthetic Metals 19, Seite 415 (1987)). Die hier erstmals synthetisierten Polycyanopyrimidine der Formel (I) ergeben z.B. mit Pyren tief dunkel gefärbte Charge-Transfer-Komplexe mit interessanten Materialeigenschaften. Darüber hinaus zeigen die Polycyanopyrimidine der Formel (I) z.T. biozide, insbesondere insektizide und fungizide Wirkungen.

Beispiel 1

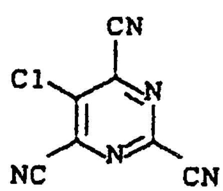

Die Lösung von 16,85 g (0,1 Mol) 5-Chloro-2,4,6-trifluoro-pyrimidin in 170 ml trockenem Dimethylformamid wird unter Rühren und unter Feuchtigkeitsausschluß bei etwa -40°C portionsweise mit 14,7 g (0,3 Mol) Natriumcyanid versetzt. Das Reaktionsgemisch wird 6 Stunden bei -25°C bis -20°C gerührt. Anschließend wird es unter Rühren in eine Mischung aus 550 g Eis und 300 ml 1-normaler wäßriger Salzsäure gegossen. Nach dem Schmelzen des Eises wird der entstandene Niederschlag abfiltriert, mit Wasser gewaschen und bis zur Gewichtskonstanz getrocknet,

Man erhält 11,10 g Festprodukt, das bei 0,1 mbar bis zu einer Badtemperatur von 200°C sublimiert wird. Sublimationsrückstand: 1,35 g. Das Sublimat besteht gemäß gaschromatographischer Analyse aus reinem 5-Chloro-2,4,6-tricyano-pyrimidin ohne jeden Anteil von Tetracyanopyrimidin. Ausbeute an sublimiertem 5-Chloro-2,4,6-tricyano-pyrimidin 51,4 % der Theorie, Schmelzpunkt 167 - 168°C (aus Cyclohexan oder aus 1,2-Dichloroethan).

IR (KBr) in $cm^{-1}$: 2261, 2250, 1529, 1352, 1194, 1089, 947, 920, 873, 788.

Beispiel 2

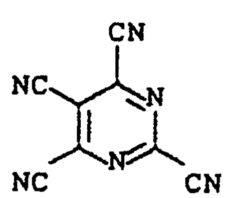

Die Lösung von 84,25 g (0,5 Mol) 5-Chloro-2,4,6-trifluoro-pyrimidin in 850 ml trockenem Dimethylformamid wird unter Rühren und unter Feuchtigkeitsausschluß bei etwa -40°C portionsweise mit 98,0 g (2 Mol) Natriumcyanid versetzt. Danach läßt man im Verlauf von etwa einer Stunde die Temperatur des Reaktionsgemisches auf +5°C ansteigen und rührt es dann weitere 6 Stunden bei +5°C bis +10°C. Anschließend wird das Reaktionsgemisch unter Einhaltung der gleichen Temperatur in eine Mischung aus 2,3 kg Eiswasser und 2 Liter 1-normale Salzsäure eingerührt. Der entstandene Niederschlag wird sofort abfiltriert, mit Eiswasser gewaschen und getrocknet.

Man erhält 43,40 g Festprodukt, das bei 0,1 mbar bis zu einer Badtemperatur von 200°C sublimiert wird. Sublimationsrückstand: 13,71 g. Das Sublimat besteht gemäß gaschromatographischer Analyse aus reinem Tetracyanopyrimidin ohne jeden Anteil von 5-Chloro-2,4,6-tricyano-pyrimidin. Ausbeute an sublimiertem Tetracyanopyrimidin 33,0 % der Theorie. Schmelzpunkt 190 - 191°C (aus Dichlormethan oder aus 1,2-Dichloroethan).

IR (KBr) in $cm^{-1}$: 2246, 1539, 1526, 1381, 934, 800, 702.

Beispiel 3

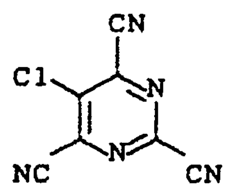

+

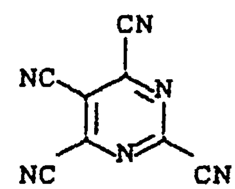

Man verfährt wie in Beispiel 2 beschrieben mit dem Unterschied, daß man das Reaktionsgemisch nach beendeter NaCN-Zugabe 6 Stunden bei -25°C bis -20°C rührt. Das Reaktionsgemisch wird wie in Beispiel 2 beschrieben aufgearbeitet; es werden 50,3 g Festprodukt erhalten, das bei 0,1 mbar bis zu einer Badtemperatur von 200°C sublimiert wird. Sublimationsrückstand: 3,47 g. Das Sublimat besteht gemäß gaschromatographischer Analyse zu 56,1 % aus 5-Chloro-2,4,6-tricyano-pyrimidin und zu 43,7 % aus Tetracyanopyrimidin. Die Gesamtausbeute an sublimierten Cyanopyrimidinen beträgt etwa 50 % der Theorie.

Beispiel 4

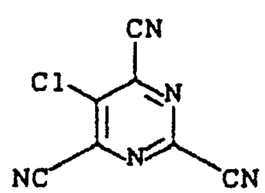

+

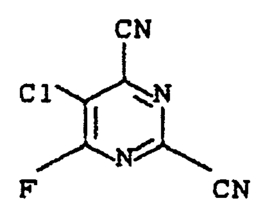

Die Lösung von 84,25 g (0,5 Mol) 5-Chloro-2,4,6-trifluoro-pyrimidin in 850 ml trockenem Dimethylformamid wird unter Rühren und unter Feuchtigkeitsausschluß bei -50°C bis -40°C portionsweise mit 66,15 g (1,35 Mol) Natriumcyanid versetzt. Danach läßt man im Verlauf von etwa einer halben Stunde die Temperatur des Reaktionsgemisches auf -30°C ansteigen und rührt es dann weitere 6 Stunden bei -30°C bis -25°C. Das Reaktionsgemisch wird unter Einhaltung der Temperatur von -30°C bis -25°C in eine Mischung aus 2,85 kg Eiswasser und 1,35 Liter 1-normale Salzsäure eingerührt; der entstandene Niederschlag wird abfiltriert, mit Wasser gewaschen und getrocknet.

Man erhält 57,60 g Festprodukt, das bei 0,1 mbar bis zu einer Badtemperatur von 180°C sublimiert wird- Sublimationsrückstand: 7,78 g. Das Sublimat besteht gemäß gaschromatographischer Analyse zu 98,2 % (entsprechend 51,6 % der Theorie) aus 5-Chloro-2,4,6-tricyano-pyrimidin und zu 1,8 % aus 5-Chloro-2,4-dicyano-6-fluoro-pyrimidin. Letztere Verbindung ist deutlich leichter flüchtig als das Hauptprodukt und scheidet sich deshalb bei der Sublimation getrennt vom Hauptprodukt an einer Stelle der Sublimationsapparatur ab, die weiter entfernt vom Heizmantel liegt als die Stelle, an der sich das Hauptprodukt abgeschieden

hat. Schmelzpunkt des 5-Chloro-2,4-dicyano-6-fluoro-pyrimidins nach dem Umkristallisieren aus Cyclohexan: 108 bis 110°C (im geschlossenen Rohr).

Nach dem Umkristallisieren des Gesamtsublimats z.B. aus 1,2-Dichloroethan wird reines 5-Chloro-2,4,6-tricyano-pyrimidin erhalten. IR (KBr) in $cm^{-1}$ von 5-Chloro-2,4-dicyano-6-fluoro-pyrimidin: 2264, 2252, 1547, 1392, 1340, 1200, 1026, 921, 779, 700.

GC-Bedingungen:

| | |
|---|---|
| Säule: | OV 1701, 20 m, 0,5 mbar Vordruck Helium |
| Programm: | 100°C - 10°C/min -300°C detektiert mit FID |

| | Retentions-Index |
|---|---|
| 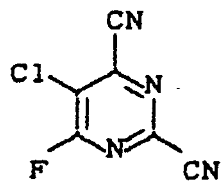 | ca. 1520 |
| 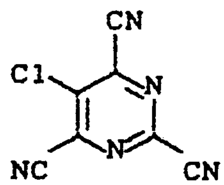 | ca. 1970 |
| 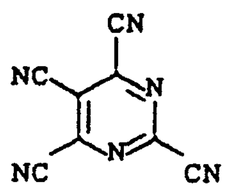 | ca. 2220 |

Beispiel 5

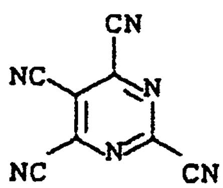

+

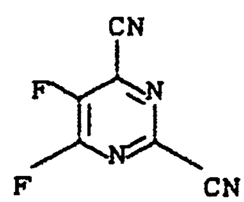

Die Lösung von 7,60 g (0,05 Mol) Tetrafluoropyrimidin in 100 ml trockenem Dimethylformamid wird unter Rühren und unter Feuchtigkeitsausschluß bei -55°C bis -50°C mit 7,35 g (0,15 Mol) Natriumcyanid versetzt. Danach läßt man die Innentemperatur auf -35°C ansteigen und rührt dann weitere 4 Stunden im Temperaturbereich von -35°C bis -30°C kräftig nach. Ohne weitere Erwärmung wird das Rohgemisch in eine Mischung aus 850 g Eiswasser und 150 ml 1 n Salzsäure eingerührt, der entstandene Niederschlag sofort abfiltriert, mit Eiswasser gewaschen und getrocknet. Das Festprodukt besteht hauptsächlich aus Tetracyanopyrimidin; die im Festprodukt enthaltenen geringen Anteile an 2,4-Dicyano-5,6-difluoro-pyrimidin können durch fraktionierte Sublimation bis 120°C/0,1 mbar isoliert werden. Schmelzpunkt 104°C.

IR (KBr) in $cm^{-1}$ von 2,4-Dicyano-5,6-difluoro-pyrimidin: 2259, 1578, 1437, 1419, 1199, 1009, 932, 804, 780, 707.

**Patentansprüche**

1. Polycyanopyrimidine der Formel

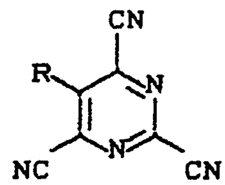

(I) ,

in der
R für Chlor oder Cyano steht.

2. Verfahren zur Herstellung von Polycyanopyrimidinen der Formel

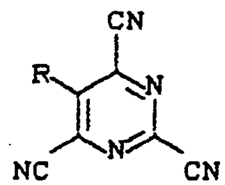

(I) ,

in der
R für Chlor oder Cyano steht,
dadurch gekennzeichnet, daß man Polyfluoropyrimidine der Formel

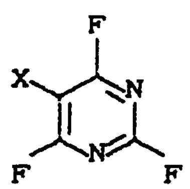

(II),

in der
X für Chlor oder Fluor steht,
mit Alkalicyaniden in organischen Verdünnungsmitteln umsetzt.

3. Verfahren nach Anspruch 2, dadurch gekennzeichnet, daß man die Umsetzung mit höchstens einem geringen Überschuß über die stöchiometrisch erforderliche Menge an Alkalicyanid vornimmt.

4. Verfahren nach Anspruch 2, dadurch gekennzeichnet, daß man die Umsetzung bei Temperaturen von -50°C bis +30°C mit der stöchiometrisch erforderlichen Menge oder einem Unterschuß an Alkalicyanid vornimmt.

Europäisches Patentamt

# EUROPÄISCHER RECHERCHENBERICHT

Nummer der Anmeldung

**EP 91 10 9245**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A,D | SYNTHETIC METALS, vol. 19, 1987, Seiten 415 - 418; A. BERLIN et al.: "Non conventional electroconductive charge transfer complexes"<br>* Seite 415, Verbindung 2 *<br>– – – | 1 | C 07 D 239/28<br>C 07 D 239/30 |
| A | CHEMICAL ABSTRACTS, vol. 94, no. 3, 19 Januar 1981 Columbus, Ohio, USA J.C. MARTIN: "Synthesis of pyridines from dicyanopyrimidines. A Diels-Alder approach to the C-ring of streptonigrin" Seite 420; ref. no. 15<br>& J. Heterocycl. Chem. 1980, 17[5], 1111-12<br>* Zusammenfassung *<br>– – – | 1 | |
| A | CHEMICAL ABSTRACTS, vol. 87, no. 21, 21 November 1977 Columbus, Ohio, USA O.P. SHKURKO et al.: "Pyrimidines. LXII. Some reactions of pyrimidine cyano derivatives" Seite 571; ref. no. 167966C<br>& Khim. Geterotsikl. Soedin. 1977, [6], 821-4<br>* Zusammenfassung *<br>– – – – – | 1 | |
| | | | **RECHERCHIERTE SACHGEBIETE (Int. Cl.5)** |
| | | | C 07 D 239/00 |

Der vorliegende Recherchenbericht wurde für alle Patentansprüche erstellt

| Recherchenort | Abschlußdatum der Recherche | Prüfer |
|---|---|---|
| Berlin | 06 September 91 | HASS C V F |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument